# FASCICULE DE BREVET EUROPEEN

(11) **EP 3 305 258 B1**
(45) Date de publication et mention de la délivrance du brevet: **08.07.2020**
(21) Numéro de dépôt: 16192207.5
(22) Date de dépôt: 04.10.2016
(51) Int. Cl.: A61F 13/00, A61K 9/00, A61K 9/70

(54) **PATCH ANTIALLERGENE**
ANTIALLERGISCHES PFLASTER
ANTI-ALLERGEN PATCH

(43) Date de publication de la demande: 11.04.2018
(73) Titulaire: The Swatch Group Research and Development Ltd, 2074 Marin (CH)
(72) Inventeur: François, Nicolas, 2000 Neuchâtel (CH); Frenkel, Erik Jan, 3210 Kerzers (CH)
(74) Mandataire: ICB SA

(56) Documents cités:
- EP-A1- 0 038 793
- EP-A1- 2 599 500
- FR-A1- 2 375 861
- US-A1- 2011 264 003

## Description

### Domaine de l'invention

L'invention se rapporte à un patch antiallergène comprenant au moins une première couche adhésive présentant une première face destinée à fixer le patch sur un article et une seconde face à laquelle est assemblée une deuxième couche barrière continue étanche à des allergènes.

### Arrière-plan de l'invention

Un tel patch est connu et est par exemple décrit dans le brevet EP 0 038 793. Il est décrit un objet en matériau flexible, tel qu'un bracelet de montre, portant un patch formé d'une couche barrière continue empêchant le passage des allergènes et/ou des irritants du matériau sur et dans la peau du porteur, et d'une couche adhésive. La couche barrière peut se présenter sous la forme d'une matrice chargée de particules métalliques ou une couche de métal en aluminium ou en argent. L'épaisseur de la couche métallique est comprise entre 150 µm et 500 µm, en particulier de 200 µm à 350 µm, de sorte que le patch est relativement épais et peu souple. De plus, dans le cas d'une couche métallique, celle-ci est directement au contact de la peau, ce qui peut être désagréable pour le porteur du bracelet. Dans le cas d'une couche barrière comprenant des particules métalliques, la couche barrière n'est pas totalement étanche à la migration des ions nickel par exemple, que l'on peut trouver notamment dans le matériau d'un fond de boite de montre. En conséquence, ce type de patch décrit dans le brevet EP 0 038 793 pour être fixé sur un bracelet de montre en cuir n'est pas adapté pour être fixé sur le fond métallique d'une boite de montre par exemple.

Une variante du patch décrit dans le brevet EP 0 038 793 a été commercialisée et comprend également une couche adhésive et une couche barrière. Ladite couche barrière est un cuir revêtu d'une couche de polyuréthane faisant office de couche barrière. Là encore, le patch obtenu a une épaisseur totale très importante de plus de 2 mm. De ce fait, lorsque ce patch est utilisé sous le fond d'une boite de montre, l'épaisseur importante du patch entraine des problèmes esthétiques au porter : la montre est trop haute sur le poignet et le patch est visible lorsque la montre est portée. De plus, le porter peut devenir inconfortable. En outre, le cuir utilisé est un cuir tanné au chrome, ce qui représente un risque potentiel de migration de chrome libre entrainant un autre risque de réaction allergique si le porteur est allergique au chrome.

### Résumé de l'invention

L'invention a notamment pour objectif de pallier les différents inconvénients des patchs antiallergènes connus.

Plus précisément, un objectif de l'invention est de fournir un patch antiallergène présentant des propriétés de barrière totalement étanche aux allergènes tout en alliant des propriétés de confort au porter.

A cet effet, la présente invention concerne un patch antiallergène comprenant au moins une première couche adhésive présentant une première face destinée à fixer le patch sur un article et une seconde face à laquelle est assemblée une deuxième couche barrière continue étanche à des allergènes.

Selon l'invention, la deuxième couche barrière comprend un film plastique métallisé, une couche de polymères nanostructurés ou un film plastique comprenant un revêtement céramique ou est une couche en métal ou en alliage métallique.

Selon l'invention le patch antiallergène comprend une troisième couche réalisée en un matériau respirant, agencé pour absorber la vapeur d'eau et sécher rapidement, ladite troisième couche étant destinée à être au contact de la peau d'un porteur de l'article, et une quatrième couche de liaison disposée entre les deuxième et troisième couches rendant solidaires lesdites deuxième et troisième couches, ledit patch présentant une épaisseur totale comprise entre 0.3 mm et 0.8 mm et l'épaisseur de la deuxième couche barrière étant inférieure à 50 µm.

Ainsi, le patch présente une couche externe confortable au porter destinée à être au contact de la peau du porteur de l'article, permettant d'apporter à la peau la respirabilité dont elle a besoin, tout en garantissant une barrière totalement étanche aux allergènes.

La présente invention concerne également l'utilisation d'un patch antiallergène tel que défini ci-dessus sur un fond de boîte d'une boite de montre.

La présente invention concerne également un procédé de fabrication d'un patch antiallergène tel que défini ci-dessus, et comprenant les étapes de :
- assembler une deuxième couche barrière à une première couche adhésive,
- assembler une troisième couche réalisée en un matériau respirant à la deuxième couche barrière au moyen d'une quatrième couche de liaison disposée entre ladite deuxième couche barrière et ladite troisième couche, ledit patch présentant une épaisseur totale comprise entre 0.3 mm et 0.8 mm et l'épaisseur de la deuxième couche barrière étant inférieure à 50 µm.

### Description sommaire des dessins

D'autres particularités et avantages ressortiront clairement de la description qui en est faite ci-après, à titre indicatif et nullement limitatif, en référence aux dessins annexés, dans lesquels :

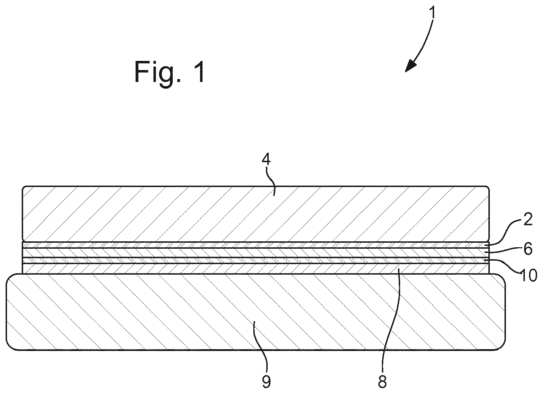

- la figure 1 est une vue en coupe schématique d'un patch selon l'invention mis en place sur le fond d'une boite de montre porté par un utilisateur.

### Description détaillée des modes de réalisation préférés

La présente invention concerne un patch antiallergène destiné à être fixé sur un article porté par un utilisateur au contact de sa peau et agencé pour faire office de barrière aux allergènes présents dans l'article en empêchant le passage des allergènes présents dans le matériau de l'article sur et dans la peau du porteur. Dans la description qui suit, un tel article est une boite de montre comprenant un fond de boite de montre sur lequel est positionné le patch antiallergène. L'article peut également être un bracelet ou tout autre bijou ou équipement électronique portable susceptible d'être en contact direct avec la peau de l'utilisateur. Le fond d'une boite de montre peut comprendre par exemple du nickel ou du cobalt qui peuvent constituer des substances allergènes et qui risquent de provoquer des allergies lorsqu'elles migrent à la surface du fond de boite pour se trouver au contact de la peau de l'utilisateur. Il est aussi possible que la sueur de certains utilisateurs en contact régulier et direct avec le métal du fond de boite le corrode et libère des allergènes.

En référence à la figure 1, le patch antiallergène 1 comprend une première couche adhésive 2 présentant une première face destinée à fixer le patch 1 sur le fond d'une boite de montre 4 et une seconde face solidaire d'une deuxième couche barrière 6 continue étanche à des allergènes, tels que le nickel ou le cobalt qui peuvent être présents dans le matériau du fond de boite de montre 4.

Selon l'invention, le patch 1 comprend également une troisième couche 8 réalisée en un matériau respirant, agencé pour absorber la vapeur d'eau générée au niveau de la peau 9 du porteur et sécher rapidement, ladite troisième couche 8 étant destinée à être au contact du porteur de l'article, et constituant donc une couche externe. Les deuxième 6 et troisième 8 couches sont rendues solidaires au moyen d'une quatrième couche de liaison 10 disposée entre lesdites deuxième 6 et troisième 8 couches.

De préférence, la troisième couche 8 peut être réalisée en microfibres ultra-fines non-tissées de polyamide, enchevêtrées ensemble par aiguilletage par exemple et imprégnées de polyuréthane. De préférence, les microfibres ont un diamètre inférieur à 0.1 µm, de préférence inférieur à 0.08 µm, et plus préférentiellement inférieur ou égal à 0.05 µm.

De telles microfibres sont respirantes, c'est-à-dire perméables à la vapeur d'eau. Elles permettent un excellent transfert d'humidité. De ce fait, la vapeur d'eau telle que la transpiration est absorbée. De telles microfibres sont également perméables à l'air et permettent une excellente circulation d'air de sorte que la troisième couche 8 sèche très rapidement pour éviter le contact humide mouillé avec la peau du porteur. Le porter est donc très confortable.

Selon une autre variante de réalisation, le matériau respirant de la troisième couche 8 peut être une membrane microporeuse constituée d'une couche ultra-fine de polytétrafluoroethylène expansé, de type GORE-TEX®.

Selon une variante, en particulier lorsque la troisième couche est une microfibre, la deuxième couche barrière 6 peut être une couche en métal ou en alliage métallique non allergène, à base de titane ou d'aluminium par exemple.

Selon une autre variante, la deuxième couche barrière 6 comprend un film plastique mince métallisé. Une telle couche est obtenue par exemple à partir d'un film polyester métallisé par évaporation par exemple. On peut utiliser avantageusement un film de polytéréphtalate d'éthylène (PET) métallisé par PACVD (Dépôt Chimique en phase Vapeur, Assisté Plasma).

Selon encore une autre variante, la deuxième couche barrière 6 peut comprendre une couche de polymères nanostructurés utilisant des nanocharges inorganiques afin de rendre la couche étanche à la diffusion de gaz ou d'ions.

Selon encore une autre variante, en particulier lorsque la troisième couche 8 est une membrane de type GORE-TEX®, la deuxième couche barrière 6 peut comprendre un film plastique mince comprenant un revêtement céramique. On peut utiliser avantageusement un film de polytéréphtalate d'éthylène (PET) comprenant un revêtement d'oxyde d'aluminium. Un film de polytéréphtalate d'éthylène revêtu d'oxyde d'aluminium et une membrane de type GORE-TEX® ont l'avantage d'être transparents. De ce fait, la combinaison d'une deuxième couche barrière comprenant un film plastique avec un revêtement céramique et d'une troisième couche en un matériau respirant constitué d'une membrane microporeuse de polytétrafluoroethylène expansé, et plus particulièrement la combinaison d'une couche barrière constituée d'un film de polytéréphtalate d'éthylène avec un revêtement d'oxyde d'aluminium et d'une troisième couche constituée d'une membrane de type GORE-TEX®, permet d'obtenir un patch complètement transparent, en choisissant également une première couche adhésive et une quatrième couche de liaison transparentes. Le patch selon l'invention est alors invisible une fois mis en place, ce qui permet de garder l'esthétique de l'article, et en particulier du fond de boite de montre, inchangée.

Avantageusement, l'épaisseur de la deuxième couche barrière 6 est inférieure à 50 µm, de préférence inférieure à 20 µm, et plus préférentiellement inférieure ou égale à 15 µm. Une épaisseur très fine est obtenue notamment en utilisant un film plastique métallisé, pour lequel l'épaisseur de la couche métallique en elle-même est inférieure à 5 µm. Ce dernier permet également d'obtenir un patch particulièrement souple.

Le patch selon l'invention présente une épaisseur totale comprise entre 0.3 mm et 0.8 mm. Il est donc très mince et peut se porter en toute discrétion sous le fond de boite de montre.

La deuxième couche barrière 6 et la troisième couche 8 sont rendues solidaires au moyen de la quatrième couche de liaison 10, qui peut être avantageusement une couche adhésive forte irréversible.

D'une manière avantageuse, la première couche adhésive 2 est à base d'un adhésif repositionnable et comprend une pellicule de protection détachable. Un tel adhésif repositionnable permet avantageusement d'enlever et remettre le patch autant de fois que nécessaire sans laisser de résidus sur le fond de boite de montre.

Selon une première variante, l'adhésif repositionnable peut être un adhésif sensible à la pression (pressure sensitive adhesive PSA) formulé par exemple à partir d'une résine acrylique afin d'avoir une adhésion forte ou, de préférence, à partir d'une résine silicone ou de tout autre adhésif exempt de substance pouvant provoquer des réactions cutanées.

Selon une deuxième variante, l'adhésif repositionnable peut être un film en silicone micro-structuré du type Gecko® de chez Nanoplast®.

Avantageusement, la troisième couche 8, en particulier lorsqu'elle est en microfibre, peut comprendre un décor réalisé par traitement laser par exemple ou par déformation du matériau de la troisième couche par marquage à chaud par exemple. Le traitement laser et le marquage à chaud permettent de créer des décors sur la troisième couche en évitant d'apporter des matières colorantes risquant d'entrainer des allergies.

Avantageusement, le matériau de la troisième couche 8, en particulier lorsqu'il s'agit de microfibres, peut être imprégné d'une substance choisie parmi le groupe comprenant des agents anti-salissures, des agents actifs cosmétiques de la peau, des agents antibactériens et des agents absorbeurs d'odeurs. Les agents anti-salissures sont par exemple des silanes fluorés permettant d'apporter un effet déperlant. Les agents actifs cosmétiques de la peau peuvent être choisis pour renforcer le film hydro-lipidique de la peau. Les agents antibactériens et absorbeurs d'odeurs peuvent être des zéolithes fonctionnalisées avec des ions cuivre, argent ou zinc permettant d'éviter le développement des bactéries responsables d'irritations cutanées et de mauvaises odeurs. Ces substances peuvent être imprégnées dans le matériau de la troisième couche en bain ou par traitement par CO₂ supercritique.

Le patch selon l'invention est fabriqué en assemblant les différentes couches décrites ci-dessus. Le procédé comprend de préférence une étape de réalisation de la deuxième couche barrière 6 par métallisation d'un film plastique, et notamment par métallisation par PACVD (Dépôt Chimique en phase Vapeur, Assisté Plasma) d'un film de polytéréphtalate d'éthylène (PET).

Le procédé peut également comprendre une étape de décoration de la troisième couche par traitement laser ou marquage à chaud.

Le procédé peut également comprendre une étape d'imprégnation du matériau de la troisième couche 8 par bain ou par traitement par CO₂ supercritique avec une substance choisie parmi le groupe comprenant des agents anti-salissures, des agents actifs cosmétiques de la peau, des agents antibactériens et des agents absorbeurs d'odeurs.

On obtient un patch très mince et très souple, très confortable au porter, permettant d'apporter à la peau du porteur la respirabilité dont elle a besoin, tout en garantissant une barrière totalement étanche aux allergènes.

Bien entendu, la présente divulgation ne se limite pas aux exemples illustrés et est susceptible de diverses variantes et modifications qui apparaîtront de manière évidente à l'homme du métier.

## Revendications

1. Patch antiallergène (1) comprenant au moins une première couche adhésive (2) présentant une première face destinée à fixer le patch sur un article et une seconde face à laquelle est assemblée une deuxième couche barrière (6) continue étanche à des allergènes, **caractérisé en ce que** la deuxième couche barrière (6) comprend un film plastique métallisé, une couche de polymères nanostructurés ou un film plastique comprenant un revêtement céramique ou est une couche en métal ou en alliage métallique, et **caractérisé en ce qu'**il comprend une troisième couche (8) réalisée en un matériau respirant, agencé pour absorber la vapeur d'eau et sécher rapidement, ladite troisième couche (8) étant destinée à être au contact de la peau d'un porteur de l'article, et une quatrième couche de liaison (10) disposée entre les deuxième (6) et troisième (8) couches rendant solidaires lesdites deuxième (6) et troisième (8) couches, ledit patch présentant une épaisseur totale comprise entre 0.3 mm et 0.8 mm et l'épaisseur de la deuxième couche barrière (6) étant inférieure à 50 µm.

2. Patch antiallergène (1) selon la revendication 1, **caractérisé en ce que** le matériau respirant de la troisième couche (8) est à base de microfibres ultra-fines non-tissées de polyamide et imprégnées de polyuréthane.

3. Patch antiallergène (1) selon la revendication 2, **caractérisé en ce que** les microfibres ont un diamètre inférieur à 0. 1µm, de préférence inférieur à 0.08 µm, et plus préférentiellement inférieur ou égal à 0.05 µm.

4. Patch antiallergène (1) selon la revendication 1, **caractérisé en ce que** le matériau respirant de la troisième couche (8) est une membrane microporeuse de polytétrafluoroethylène expansé.

5. Patch antiallergène (1) selon la revendication 4, **caractérisé en ce que** la deuxième couche barrière (6) comprend le film plastique comprenant un revêtement céramique et **en ce que** le matériau respirant de la troisième couche (8) est la membrane microporeuse de polytétrafluoroethylène expansé.

6. Patch antiallergène (1) selon l'une des revendications précédentes, **caractérisé en ce que** l'épaisseur de la deuxième couche barrière (6) est inférieure à 20 µm, et plus préférentiellement inférieure ou égale à 15 µm.

7. Patch antiallergène (1) selon l'une des revendications précédentes, **caractérisé en ce que** la première couche adhésive (2) est à base d'un adhésif repositionnable et comprend une pellicule de protection détachable.

8. Patch antiallergène (1) selon l'une des revendications précédentes, **caractérisé en ce que** la troisième couche (8) comprend un décor réalisé par traitement laser ou par marquage à chaud.

9. Patch antiallergène (1) selon l'une des revendications précédentes, **caractérisé en ce que** le matériau de la troisième couche (8) est imprégné d'une substance choisie parmi le groupe comprenant des agents anti-salissures, des agents actifs cosmétiques de la peau, des agents antibactériens et des agents absorbeurs d'odeurs.

10. Utilisation d'un patch antiallergène (1) selon l'une des revendications 1 à 9 sur un fond de boite d'une boite de montre (4).

11. Procédé de fabrication d'un patch antiallergène (1) selon l'une des revendications 1 à 9, **caractérisé en ce qu'**il comprend les étapes de :
- assembler une deuxième couche barrière (6) à une première couche adhésive (2)
- assembler une troisième couche (8) réalisée en un matériau respirant à la deuxième couche barrière (6) au moyen d'une quatrième couche de liaison (10) disposée entre ladite deuxième couche barrière (6) et ladite troisième couche (8), ledit patch présentant une épaisseur totale comprise entre 0.3 mm et 0.8 mm et l'épaisseur de la deuxième couche barrière (6) étant inférieure à 50 µm.

12. Procédé de fabrication d'un patch antiallergène (1) selon la revendication 11, **caractérisé en ce qu'**il comprend une étape de réalisation de la deuxième couche barrière (6) par métallisation d'un film plastique.

13. Procédé de fabrication d'un patch antiallergène (1) selon l'une des revendications 11 et 12, **caractérisé en ce qu'**il comprend une étape de décoration de la troisième couche (8) par traitement laser ou marquage à chaud.

14. Procédé de fabrication d'un patch antiallergène (1) selon l'une des revendications 11 à 13, **caractérisé en ce qu'**il comprend une étape d'imprégnation du matériau de la troisième couche (8) par bain ou par traitement par CO₂ supercritique avec une substance choisie parmi le groupe comprenant des agents anti-salissures, des agents actifs cosmétiques de la peau, des agents antibactériens et des agents absorbeurs d'odeurs.

## Patentansprüche

1. Antiallergisches Pflaster (1), umfassend mindestens eine erste Klebstoffschicht (2) mit einer ersten Fläche, die dazu bestimmt ist, das Pflaster an einem Gegenstand zu befestigen, und einer zweiten Fläche, auf der eine zweite, durchgehende Barriereschicht (6) angebracht ist, die gegenüber Allergenen dicht ist, **dadurch gekennzeichnet, dass** die zweite Barriereschicht (6) einen metallisierten Kunststofffilm, eine nanostrukturierte Polymerschicht oder einen Kunststofffilm umfasst, der eine keramische Beschichtung aufweist oder eine Schicht aus Metall oder aus einer Metalllegierung ist, und **dadurch gekennzeichnet, dass** es eine dritte Schicht (8) umfasst, die aus einem atmungsaktiven Material hergestellt ist und so beschaffen ist, dass sie Wasserdampf absorbiert und schnell trocknet, wobei die dritte Schicht (8) dazu bestimmt ist, mit der Haut eines Trägers des Gegenstands in Kontakt zu sein, und eine vierte Verbindungsschicht (10) umfasst, die zwischen der zweiten (6) und der dritten (8) Schicht angeordnet ist und die zweite (6) und die dritte (8) Schicht aneinander befestigt, wobei das Pflaster eine Gesamtdicke im Bereich von 0,3 mm bis 0,8 mm aufweist und die Dicke der zweiten Barriereschicht (6) kleiner als 50 µm ist.

2. Antiallergisches Pflaster (1) nach Anspruch 1, **dadurch gekennzeichnet, dass** das atmungsaktive Material der dritten Schicht (8) auf der Basis nicht gewebter, ultrafeiner und mit Polyurethan imprägnierter Polyamid-Mikrofasern hergestellt ist.

3. Antiallergisches Pflaster (1) nach Anspruch 2, **dadurch gekennzeichnet, dass** die Mikrofasern einen Durchmesser haben, der kleiner als 0,1 um, vorzugsweise kleiner als 0,08 µm und besonders bevorzugt kleiner oder gleich 0,05 µm ist.

4. Antiallergisches Pflaster (1) nach Anspruch 1, **dadurch gekennzeichnet, dass** das atmungsaktive Material der dritten Schicht (8) eine mikroporöse Membran aus expandiertem Polytetrafluorethylen ist.

5. Antiallergisches Pflaster (1) nach Anspruch 4, **dadurch gekennzeichnet, dass** die zweite Barriereschicht (6) einen Kunststofffilm umfasst, der eine Keramikbeschichtung aufweist, und dass das atmungsaktive Material der dritten Schicht (8) die mikroporöse Membran aus expandiertem Polytetrafluorethylen ist.

6. Antiallergisches Pflaster (1) nach einem der vorhergehenden Ansprüche, **dadurch gekennzeichnet, dass** die Dicke der zweiten Barriereschicht (6) kleiner als 20 µm ist und besonders bevorzugt kleiner oder gleich 15 µm ist.

7. Antiallergisches Pflaster (1) nach einem der vorhergehenden Ansprüche, **dadurch gekennzeichnet, dass** die erste Klebstoffschicht (2) auf Basis eines neu positionierbaren Klebstoffs hergestellt ist und eine abziehbare Schutzfolie umfasst.

8. Antiallergisches Pflaster (1) nach einem der vorhergehenden Ansprüche, **dadurch gekennzeichnet, dass** die dritte Schicht (8) ein durch Laserbearbeitung oder Heißprägen hergestelltes Dekor aufweist.

9. Antiallergisches Pflaster (1) nach einem der vorhergehenden Ansprüche, **dadurch gekennzeichnet, dass** das Material der dritten Schicht (8) mit einer Substanz imprägniert ist, die aus der Gruppe ausgewählt ist, die schmutzabweisende Wirkstoffe, kosmetisch aktive Hautwirkstoffe, antibakterielle Wirkstoffe und geruchsabsorbierende Wirkstoffe umfasst.

10. Verwendung eines antiallergischen Pflasters (1) nach einem der Ansprüche 1 bis 9 auf einem Gehäuseboden eines Uhrengehäuses (4).

11. Verfahren zum Herstellen eines antiallergischen Pflasters (1) nach einem der Ansprüche 1 bis 9, **dadurch gekennzeichnet, dass** es die folgenden Schritte umfasst:
- Zusammenfügen einer zweiten Barriereschicht (6) und einer ersten Klebstoffschicht (2),
- Zusammenfügen einer dritten Schicht (8), die aus einem atmungsaktiven Material hergestellt ist, und der zweiten Barriereschicht (6) mittels einer vierten Verbindungsschicht (10), die zwischen der zweiten Barriereschicht (6) und der dritten Schicht (8) angeordnet ist, wobei das Pflaster eine Gesamtdicke im Bereich von 0,3 mm bis 0,8 mm aufweist und die Dicke der zweiten Barriereschicht (6) kleiner als 50 µm ist.

12. Verfahren zum Herstellen eines antiallergischen Pflasters (1) nach Anspruch 11, **dadurch gekennzeichnet, dass** es einen Schritt zum Herstellen der zweiten Barriereschicht (6) durch Metallisieren eines Kunststofffilms umfasst.

13. Verfahren zum Herstellen eines antiallergischen Pflasters (1) nach einem der Ansprüche 11 und 12, **dadurch gekennzeichnet, dass** es einen Schritt zum Dekorieren der dritten Schicht (8) durch Laserbehandlung oder Heißprägen umfasst.

14. Verfahren zum Herstellen eines antiallergischen Pflasters (1) nach einem der Ansprüche 11 bis 13, **dadurch gekennzeichnet, dass** es einen Schritt zum Imprägnieren des Materials der dritten Schicht (8) mittels eines Bades oder mittels einer Behandlung mit überkritischem CO₂ mit einer Substanz umfasst, die aus der Gruppe gewählt ist, die schmutzabweisende Wirkstoffe, kosmetisch aktive Hautwirkstoffe, antibakterielle Wirkstoffe und geruchsabsorbierende Wirkstoffe enthält.

## Claims

1. Antiallergen patch (1) comprising at least a first, adhesive layer (2) having a first surface intended to fasten the patch to an article and a second surface to which a second, continuous allergen-proof barrier layer (6) is attached, **characterised in that** the second, barrier layer (6) comprises a metallised plastic film, a layer of nanostructured polymers or a plastics film comprising a ceramic coating or is a metal or metal-alloy layer, and **characterised in that** it comprises a third layer (8) made of a breathable material, designed to absorb water vapour and dry quickly, said third layer (8) being intended to be in contact with the skin of a person wearing the article, and a fourth, bonding layer (10), which is placed between the second (6) and third (8) layers and secures said second (6) and third (8) layers to one another, said patch having a total thickness of between 0.3 mm and 0.8 mm and the thickness of the second, barrier layer (6) being less than 50 µm.

2. Antiallergen patch (1) according to claim 1, **characterised in that** the breathable material of the third layer (8) is based on ultrafine nonwoven polyamide microfibres impregnated with polyurethane.

3. Antiallergen patch (1) according to claim 2, **characterised in that** the microfibres have a diameter of less than 0.1 µm, preferably of less than 0.08 µm, and more preferably of less than or equal to 0.05 µm.

4. Antiallergen patch (1) according to claim 1, **characterised in that** the breathable material of the third layer (8) is a microporous membrane of expanded polytetrafluoroethylene.

5. Antiallergen patch (1) according to claim 4, **characterised in that** the second, barrier layer (6) comprises the plastics film comprising a ceramic coating, and **in that** the breathable material of the third layer (8) is the microporous membrane of expanded polytetrafluoroethylene.

6. Antiallergen patch (1) according to any of the preceding claims, **characterised in that** the thickness of the second, barrier layer (6) is less than 20 µm, and more preferably less than or equal to 15 µm.

7. Antiallergen patch (1) according to any of the preceding claims, **characterised in that** the first, adhesive layer (2) is based on a repositionable adhesive and comprises a detachable protective film.

8. Antiallergen patch (1) according to any of the preceding claims, **characterised in that** the third layer (8) comprises a decoration produced by laser processing or hot stamping.

9. Antiallergen patch (1) according to any of the preceding claims, **characterised in that** the material of the third layer (8) is impregnated with a substance chosen from the group comprising antisoiling agents, cosmetic active agents for the skin, antibacterial agents and odour-absorbing agents.

10. Use of an antiallergen patch (1) according to any of claims 1 to 9 on a case back of a watch case (4).

11. Method for manufacturing an antiallergen patch (1) according to any of claims 1 to 9, **characterised in that** it comprises the steps of:
- attaching a second, barrier layer (6) to a first, adhesive layer (2)
- attaching a third layer (8) made of a breathable material to the second, barrier layer (6) by means of a fourth, bonding layer (10) placed between said second, barrier layer (6) and said third layer (8), said patch having a total thickness of between 0.3 mm and 0.8 mm and the thickness of the second, barrier layer (6) being less than 50 µm.

12. Method for manufacturing an antiallergen patch (1) according to claim 11, **characterised in that** it comprises a step of producing the second, barrier layer (6) by metallising a plastics film.

13. Method for manufacturing an antiallergen patch (1) according to either claim 11 or claim 12, **characterised in that** it comprises a step of decorating the third layer (8) by laser processing or hot stamping.

14. Method for manufacturing an antiallergen patch (1) according to any of claims 11 to 13, **characterised in that** it comprises a step of impregnating the material of the third layer (8), in a bath or by supercritical CO₂ treatment, with a substance chosen from the group comprising antisoiling agents, cosmetic active agents for the skin, antibacterial agents
